# EUROPEAN PATENT SPECIFICATION

(11) **EP 1 845 914 B1**
(45) Date of publication and mention of the grant of the patent: **09.09.2009**
(21) Application number: 06701070.2
(22) Date of filing: 05.01.2006
(51) Int. Cl.: A61F 7/00

(54) **APPARATUS FOR PROVIDING A HEATING OR COOLING EFFECT**
GERÄT ZUR BEREITSTELLUNG EINES WÄRMENDEN ODER KÜHLENDEN EFFEKTS
APPAREIL FOURNISSANT UN EFFET DE CHAUFFAGE ET DE REFROIDISSEMENT

(30) Priority: 13.01.2005 GB 0500630
(43) Date of publication of application: 24.10.2007
(73) Proprietor: Swellaway Ltd., Ashopton Bamford, Hope Valley Derbyshire S33 0BY (GB)
(72) Inventor: DULAKE, Nick, Sheffield S1 1 WB (GB); MILLS, Richard, Derbyshire S33 0BY (GB)
(74) Representative: Jackson, Nicholas Andrew
(86) International application number: PCT/GB2006/000032
(87) International publication number: WO 2006/075134

(56) References cited:
- WO-A-00/40186
- US-A- 5 800 490
- US-A1- 2003 144 619

## Description

### Field of the Invention

The present invention relates to a device configured to provide a heating and cooling effect to a human or animal, and in particular although not exclusively, to a device for treating a damaged human or animal limb or joint.

### Background to the Invention

The recommended treatment for common sprain, strain or impact injuries to joints, ligaments, tendons and muscles involves cooling of the injured region. One of the most primitive forms of treatment of such injuries is the application of a bag of ice or frozen matter applied directly to the effected body part.

In order to assist the natural healing process, compression may also be applied to the injured region in addition to the alternation of heating and cooling.

A number of devices have been proposed in the art directed to the treatment of injured joints and the like.

GB 2202447 discloses apparatus for cooling a limb of an animal comprising a thermoelectric module working on the Peltier principle. The thermoelectric module is secured to the injured limb to be cooled at a first surface with a heat sink being positioned at a second surface configured to dissipate heat extracted from the first surface. The thermoelectric module is powered by a remotely positioned power source.

US 5,800,490 discloses a modular lightweight heating and cooling device configured for the treatment of injured human or animal body parts. The device comprises a strap for positioning around a limb having mounting means formed within the strap configured to house as plurality of Peltier elements. Each Peltier element is situated between two conducting copper plates with a heat sink fan positioned adjacent one face. The Peltier element provides a heating or cooling effect to the injured region via a sealed gel pack positioned between a copper conducting plate and the skin of the human or animal. A battery pack, powering the Peltier assembly may be positioned at the strap or remotely located.

US 6,023,932 discloses a portable topically cooling device for an animal or human configured to relieve pain and swelling from injured joints, muscles or the like. The device comprises a thermoelectric unit having a cold side and a warm side, a DC source connected to the thermoelectric unit and a heat sink mounted at the warm side of the unit. A strap is provided for securing the device to the human or animal. A cushion material is provided at the cooling face of the unit for positioning against the skin of the animal or human.

US 4,470,263 discloses a plurality of Peltier cells attached to a garment with the cold surface of the Peltier cell in thermal contact with the skin of the patient via intermediate positioned layers of a metalised cloth and a foam rubber layer. Heat pipes positioned adjacent to the Peltier cell assist in the transfer of heat away from the person via the Peltier cell.

WO 00/40186 discloses a device to treat the tamporomandibular and maxilla mandibular region of a patient with cryo-thermal and/or compression therapy. The device, in the form of a head scarf, comprises a heating component having a plurality of individual heating elements, and ari inflatable layer containing a plurality of sacks capable of inflation by an inflation fluid configured to move freely between the sacks. A plurality of hard plates, housed within a fabric layer are configured to resist bulging of the inflatable layer when inflated.

Whilst these prior art therapeutic devices are advantages over more primitive, conventional treatment apparatus such as gel treatment packs and the like, the inventors have identified a number of disadvantages.

In particular, the contact area of the thermoelectric modules with the skin region of the patient is limited to approximately the surface area of each Peltier device. Additionally, the form and amount of compression provided by known devices is limited and may be difficult to apply correctly involving adjustment and re-adjustment of attachment straps for example.

What is required therefore is a device configured for treating a damages region of a human or animal which addresses the above disadvantages.

### Summary of the Invention

The inventors provide a device configured to treat a human or animal body part or limb by providing a heating and/or cooling effect in addition to a compressive force. The inventors have realised that in order to treat injured joints and the like as effectively as possible, the application of heat or the removal of heat (cooling effect) should be spread over as greater surface area as possible of the joint to be treated. In order to achieve this, the present invention utilises a thermally conductive material in contact with at least one thermoelectric module. Accordingly, heat generated at a surface of the thermoelectric module is conducted and distributed over the surface area of the conductive material. Similarly, when the thermoelectric module is operating so as to provide a cooling effect, in which heat is drawn from one face of the thermoelectric module and dissipated via a heat sink at an adjacent face, the resulting cooling effect propagates across the surface area of the conductive material. The present invention therefore is configured for providing a heating or cooling effect over a considerably larger surface area than similar devices found in the art.

An additional advantage with the use of a conductive material comprising a surface area of at least twice the size of the heating or cooling face of the thermoelectric module is the increased control of temperature regulation at the effected body part due to the larger thermal conducting mass. This is in contrast to prior art heating devices which use one or more thermoelectric devices to supply heat or a cooling effect directly to the skin or via a conductive material of comparable surface area to the active face of thermoelectric module. Accordingly, the need for sophisticated temperature control apparatus is avoided.

According to specie implementations of the present invention, the thermally conductive material may be pre-shaped and/or configurable for shaping to correspond to the contours of the specific human or animal body part and in particular a movable joint. As the active face of a thermoelectric module, operating under the Peltier principle is generally planar, the thermoelectric module does not necessarily need to be located directly at the injured region whereby its heating or cooling effect may be conducted to a region or regions of the conductive material that are shaped and configured for contact with the effected body part or region.

In particular, the thermoelectric module may be isolated from the skin so as to avoid the human or animal being exposed to excessively high or low operating temperatures.

The present invention may comprise a plurality of separate pieces of conductive material interconnected via one or a plurality of movable joints. Alternatively, the separate pieces of conductive material may be fixably connected together to form a conductive material of fixed structure comprising a plurality of individual interconnected pieces. Typically, the pieces may be tacked, welded, stapled, bolted, glued, riveted or slotted together.

In order to provide a compressive force to the affected body part of the present invention comprise an expandable sack in which a fluid introduced into the sack positioned against or around the effected area, serves to provide a compressive force. Further, by regulating the flow of fluid into the sack and/or controlling the amount of fluid drawn from the sack, the present invention is configured to provide a massaging effect to the effected region.

According to a first aspect of the present invention there is provided a device configured to provide a heating and a cooling effect to a region of a human or animal, said device comprising: at least one thermoelectric module capable of generating heat and said thermoelectric module also capable of providing a cooling effect; at least one solid conductive material in contact with said thermoelectric module, wherein said conductive material is capable of positioning in contact with said region of said human or animal and configured to transfer said heat and said cooling effect generated by said thermoelectric module to said human or animal; at least one expandable sack, said sack comprising a plurality of compartments, said sack capable of generating a compressive force to said region of said human or animal; and means for securing said conductive material and said expandable sack in contact with said region of said human or animal,
Characterised by further comprising
Said plurality of compartments are separate to each other and are each independently controllable such that the preserve in one compartment can be configured to be different to the pressure in another said compartment.

The device further comprises control means configured to control the heating and cooling effect provided by the thermoelectric module in addition to control and regulation of the flow of fluid into and/or out of the expandable sack. The control means may be positioned remote from the thermoelectric module and/or the means for securing the conductive material at the effected region. The control means may comprise any known control apparatus found in the art including conventional circuit boards and the like to enable a user to set and control the operative temperature output of the thermoelectric module.

The expandable sack is configurable for use with any fluid, in particular a cryogenic fluid, a liquid, a gel, a gas being in particular air. The expandable sack, capable of being expanded by the fluid, may comprise a single internal fluid chamber or may be divided into a plurality of channels or a network or compartments in fluid communication with one another. Accordingly, by dividing the internal fluid chamber of the expandable sack into a plurality of separate compartments, the expandable sack may be configured to provide a non-uniform compressive force via its surface area positioned opposed to the skin of the human or animal. That is, the pressure of the fluid within the internal channels or compartments may be regulated and controlled independently whereby the pressure in one compartment may differ from the pressure in a second compartment. Accordingly, the channels or compartments may be isolated from one another, the internal pressure of which being controlled independently. The device of the present invention is therefore configured to provide simultaneously a plurality of compressive forces to the affected body part, the compressive forces being of different magnitudes as desired.

The control means for regulating the amount of fluid introduced into said sack can provide a massaging action to said human or animal.

Optionally, the device may further comprise an additional bladder capable of housing a conductive liquid, the bladder being positioned at the thermoelectric module and/or the conductive material configured to conduct the heat or the cooling effect from the conductive material and/or thermoelectric module to the skin of the human or animal. Like the expandable sack, the bladder may comprise a single internal chamber or may be divided into a plurality of channels or network of compartments in fluid communication with one another.

The thermoelectric module may be a Peltier module comprising heat dissipation means positioned at one face of the module configured to dissipate heat drawn from a cooling face. The means for securing the conductive material and the expandable sack to the human or animal may be formed as a strap or wrap, in particular an elasticated wrap comprising a suitable fastening means such as hook and loop type fasteners.

In order for a user to monitor and set the operative temperature of the thermoelectric module, a temperature sensor may be positioned at the thermoelectric module and/or selected regions of the conductive material. The temperature sensor may be coupled with suitable temperature display means configured to indicate the working and/or pre-set temperature of the thermoelectric module. Accordingly, the control means may comprise one or more thermostats configured for regulating the operative temperature of the thermoelectric module in response to the output of the temperature sensor.

A ratio of the surface area of the conductive material capable of positioning in contact with the human or animal body part to the surface area of the active face (heating or cooling face) of the thermoelectric module is within the range 2-15: 1.

The conductive material may comprise at least two separate pieces of conductive materials joined together and movable relative to one another. Optionally, the present invention may comprise any number of a plurality of separate conductive material pieces movably joined together. The conductive material pieces may be pivotally mounted, slidably mounted, detachably mounted, riveted, pinned or hinged together to form a movable joint.

Preferably, the at least one piece of conductive material comprises one or a plurality of bent or curved regions.

Preferably, the conductive material comprises a non-uniform thickness.

Preferably, the conductive material is covered by an insulating material on one or more faces not configured for contact with the human or animal skin. The insulating material is configured to thermally insulate and enhance the heating or cooling effect provided by the conductive material. Optionally, the conductive material may be coated or covered by a fabric, a foam or polymer material.

Alternatively, the conductive material may comprise a metallic mesh or webbing.

### Brief Description of the Drawings

For a better understanding of the invention and to show how the same may be carried into effect, there will now be described by way of example only, specific embodiments, methods and processes according to the present invention with reference to the accompanying drawings in which:
Fig 1 herein illustrates the heating and cooling device according to a specific implementation of the present invention wrapped around an arm of a person;
Fig 2 herein illustrates a cross sectional side elevation view of the thermoelectric module and associated additional components of the heating and cooling device;
Fig 3 herein illustrates an exploded perspective view of the heating and cooling device in which a conductive material is divided into two pieces joined together by a movable joint;
Fig 4 herein illustrates a perspective view of the heating and cooling device further comprising an expandable sack positioned about the conductive material;
Fig 5 herein is cross sectional side elevation view of selected layers of the heating and cooling device;
Fig 6 herein is a cross sectional plan view of the expandable sack comprising a network of internal channels;
Fig 7 herein illustrates the heating and cooling device connected to suitable control means according to a specific implementation of the present invention;
Fig 8, herein illustrates schematically selected components of the heating and cooling device according to a specific implementation of the present invention;

### Detailed Description

There will now be described by way of example a specific mode contemplated by the inventors. In the following description numerous specific details are set forth in order to provide a thorough understanding. It will be apparent however, to one skilled in the art, that the present invention may be practiced without limitation to these specific details. In other instances, well known methods and structures have not been described in detail so as not to unnecessarily obscure the description.

A device is provided configured for generating heat or providing a cooling effect to a region or body part of a human or animal. The inventors utilise at least one conventional, known thermoelectric module operating according to the Peltier principle. The present invention is particularly adapted for the treatment of sprains, strains or impact injuries to joints, ligaments, tendons and muscles where the recommended treatment involves topical application of heating and cooling and/or alternate heating and cooling cycles. The effectiveness of the device, utilising at least one Peltier cell is significantly enhanced by the use of at least one thermally conductive material positioned intermediate and in thermal contact with the at least one thermoelectric module and the skin of a patient. Accordingly, the heating or cooling effect generated by the thermoelectric module is conducted or translated by the conductive material to an area of skin in contact with the material.

Additionally, the present invention comprises at least one expandable sack for positioning about the conductive material such that by introduction of a fluid into the sack, a compressive force may be applied to the body part or region to be treated.

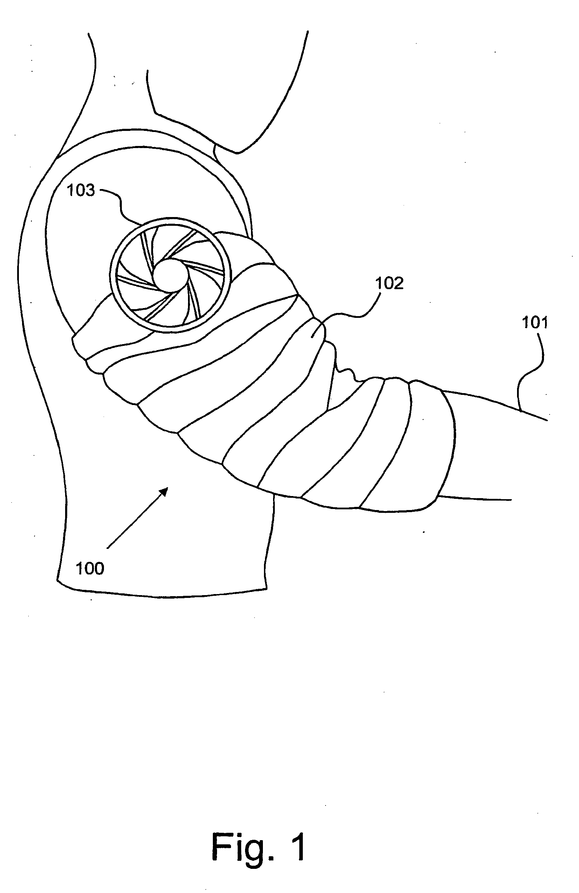

Figure 1 herein illustrates a perspective view of the device 100 wrapped around the arm of a person 101. The device comprises a component 103 configured to generate heat or provide a cooling effect to a contact region of the patient.

According to the specific implementation of the present invention, component 103 comprises at least one known Peltier cell incorporating regions of an n-type semiconductor and regions of a p-type semiconductor. On the application of current to the Peltier cell, the cell is configured to provide a heating effect or a cooling effect at an active surface according to the Peltier principle. Typical Peltier cells comprise an active surface area of approximately 6 to 6.5 cm² In order to increase this active surface area a conductive material is positioned in contact with the active face.

The device further comprises means to provide a compressive force to the region to be treated to enhance the therapeutic effect. As illustrated in figure 1 herein, a portion of the thermoelectric module, the conductive material and the means for providing a compressive force may be housed within strap 102 configured to be secured about the injured joint or region.

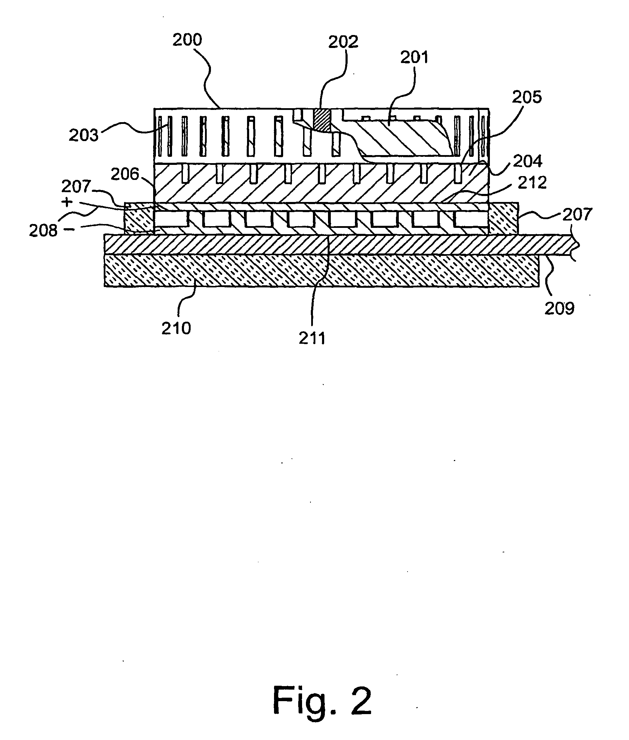

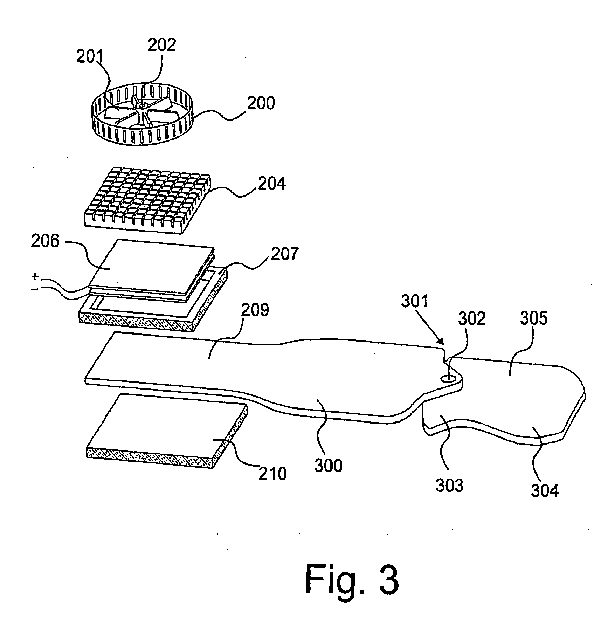

Figure 2 herein illustrates a cross sectional side elevation view of a portion of the present invention and figure 3 herein illustrates an exploded perspective view of selected components of the present invention. A main body portion 204 designed to operate as a heat sink is sandwiched between a heat dissipation fan 200 and the thermoelectric Peltier cell 206. The heat sink may comprise a suitable metallic material and has a plurality of channels 205 extending through an upper region to assist in heat dissipation from the Peltier cell 206. The heat dissipation means 200 comprises a casing comprising a plurality of vents or heat transfer fins 203. A rotatably mounted fan is housed within means 200 in which a plurality of fan blades 201 extend radially from a central spindle 202. A material 207 is located around the periphery of the Peltier cell configured to assist in maintaining the thermal gradient between the active surface of the Peltier cell 211 and an adjacent face 212. A conductive material 209 is positioned in thermal contact with the active face 211 of the Peltier cell. A non-conductive material 210 is positioned in thermal contact with the conductive material 209 in a region opposed to the active face 211 of the Peltier cell.

The Peltier cell is connected to a electrical power source using conventional wiring. In operation, heat may be omitted or absorbed at the active face 211 depending upon the direction of current flow. For example, and in a cooling mode of operation the DC current passes from the n-type to p-type semiconductive material causing a decrease in temperature at the cold junction, resulting in absorption of heat from the environment in contact with active face 211. Heat is carried through the Peltier cell by electron transport and released on the adjacent face 212. The amount of heat is proportional to the applied current and the number of p-n junctions connected electronically in series. The heat transferred to face 212 is absorbed by heat sink 204 and subsequently dissipated by the action of the rotating fan blades 201.

By reversing the current flow, electron transport across the p-n junctions is reversed such that the active face 211 becomes hot, whereby this heat is transferred to the environment in thermal contact with this active face.

The present invention utilises at least one piece of conductive material 209 in thermal contact with the active face 211 of the Peltier cell 206. Accordingly, when operating in a heating mode, heat energy is transferred to the conductive material and subsequently the skin of a patient in contact with the material. When operating in a cooling mode, heat energy is drawn from the conductive material by the Peltier cell 206 via active face 211.

The present invention utilises a conductive material comprising a significantly larger surface area than active face 211 of the Peltier cell. The conductive material may be formed from a sheet material of uniform or non-uniform thickness, the sheet material being in particular metallic copper.

According to further specific implementations of the present invention, the conductive material may comprise a metallic mesh, webbing or the like configured to allow thermal conduction.

Therapeutic treatment of the injured joint or region is most effective when substantially the entire region is subjected to the topical heating and cooling. Accordingly, the conductive material may comprise one or a plurality of bent or curved regions 300, 303, 304 configured to match the curvature and contours of the body part.

The conductive material is divided into two pieces 209, 305 joined together to form a movable joint 301 by suitable pivot means 302. For example, when secured to an arm region of a human, joint 301 is capable of synchronised movement with the elbow joint of the arm.

According to further specific implementations of the present invention the conductive sheet material may comprise a plurality of movable joints in which the pieces of conductive material are slidably and/or detachably mounted.

According to specific implementations of the present invention the conductive material 209 may be permanently or releasably positioned in contact with the Peltier cell whereby when the conductive material is releasably attached, the device is configurable for independent use with any one of a plurality of different pieces of conductive material configured and shaped specifically for positioning in contact with different regions of a human or animal body, for example an elbow joint, a neck, an ankle, a knee or lower back. Additionally, a suitable sleeve or locating/receiving means may be provided adjacent the active face of the Peltier cell configured to receive and releasably secure the conductive material in position, at the Peltier cell.

Conductive material 209, 305 may be pre-formed with a plurality of bent and curved regions and/or may be of an appropriate thickness to allow a user to manipulate the sheet material into the desired shape to more accurately correspond to the contours of an injured joint or region. In particular, where the conductive material is of non-uniform thickness, certain thinner regions of the material may allow manipulation by a user whilst thicker regions may be configured to retain a pre-formed shape and configuration.

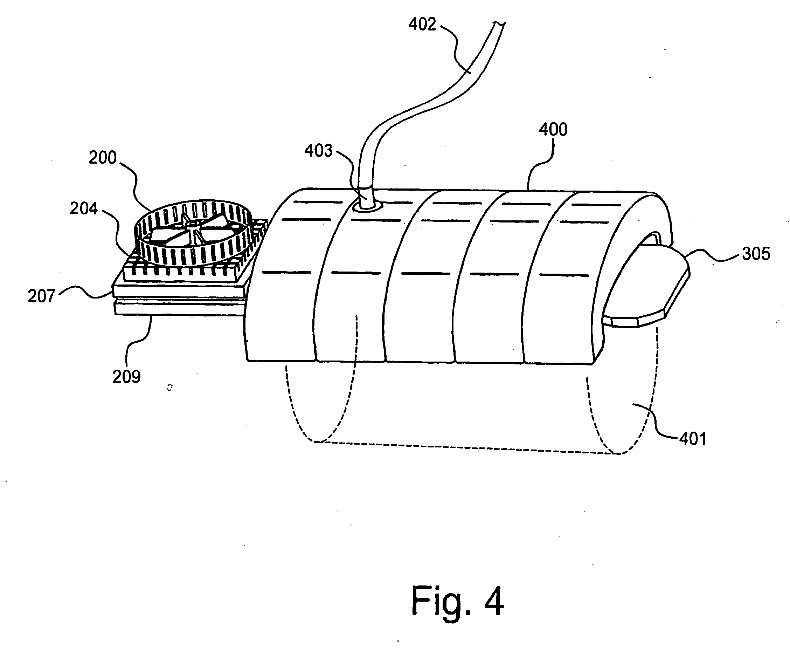

Figure 4 herein illustrates a perspective view of the device of figure 3 herein further comprising an expandable sack 400 positioned over and about the conductive material 209, 305. A suitable strap or support 401 connected to or positioned over and about the expandable sack is configured to secure the device in position. A valve 403 allows communication between the expandable sack 400 and tubing 402 enabling an expansion fluid to be introduced and expelled from the sack 400. Expandable sack 400 is configured for the temporal storage of an expansion fluid, in particular air.

When the expandable sack 400 is secured in position around the treatment region of the patient as illustrated in figure 1 herein any expansion of the sack 400 will result in a corresponding tightening of the strap 102 and/or 401 thereby providing a compressive force to the injured region. Additionally, by controlling the flow of fluid into and out of the expandable sack, a massaging effect may be achieved so as to enhance the therapeutic effect of the present invention.

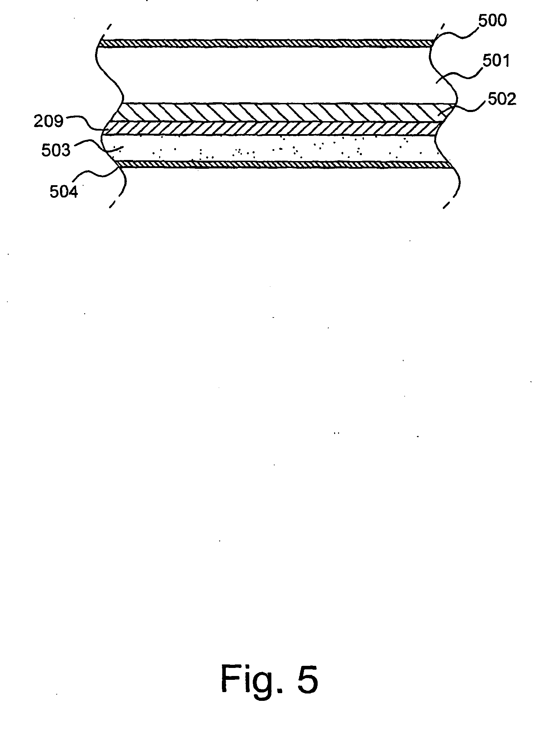

Figure 5 herein illustrates a cross sectional side elevation view through the device according to a slightly modified specific implementation of the present invention. The device comprises an outermost layer 500 corresponding to the outermost layer of strap 102 configured to substantially encase the thermoelectric cell, the conductive material and the expandable sack. The expandable sack 400 housed by the exterior layer 500 comprises a single internal chamber 501 configured for the temporal storage of the expansion fluid. A thermally insulating layer 502 is sandwiched between the expandable sack and the conductive material 209. The insulating material 502 is provided at one or more exterior faces of the conductive material, these exterior faces being in non-contact with the skin of the patient. The thermally insulating material may be provided over a portion or the entire exterior surface of the conductive material 209, 305. The thermally insulating material 502 comprises known insulating materials including ceramics, foams, polymer based materials and any organic and inorganic insulating composite. Accordingly, improved thermal stability of the conductive material is achieved in addition to a respective increase and decrease in the highest and lowest temperatures obtainable by the coating of selected regions of the conductive material with the insulating material 502. A bladder 503 is positioned adjacent the conductive material 209. Bladder 503 contains a thermally conducting fluid, in particular a gel, positioned intermediate between the skin of a patient and the conducting material 209. The presence of the conducting fluid within the bladder serves to increase thermal contact between the skin and the conductive material 209 particularly where the treatment region comprises significant surface contouring and undulations, selected regions of which may not otherwise come into contact with the conductive plate 209, 305.

The various components of the heating and cooling device are insulated by an inner most layer 504, optionally being a fabric layer similar to outermost layer 500. Layers 500, 504 provide structural support and a means for housing and retaining the various internal components in position.

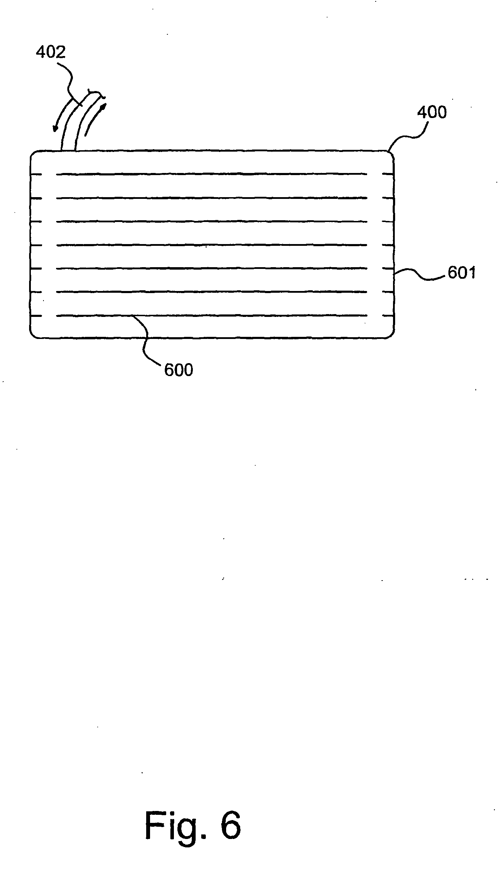

Figure 6 herein is a cross sectional plan view of a slightly modified version of the expandable sack 400 comprising a network of internal passageways being defined by a plurality of internal walls 600, 601. A substantially uniform expansion of sack 400 may be achieved using a network of internal passageways whereby non-uniform or bulging of the sack is avoided as the fluid is distributed evenly between internal walls 600, 601.

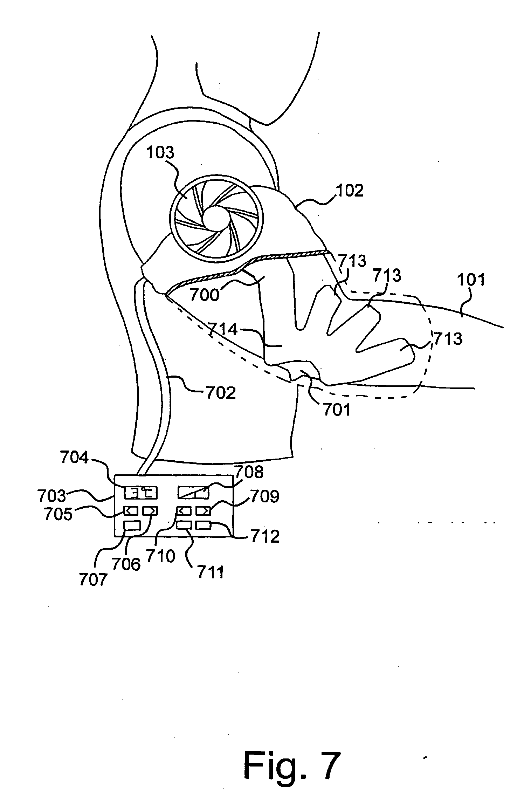

Referring to figure 7 herein there is illustrated a perspective view of the heating and cooling device of figure 1 herein wherein a portion of outer sleeve 102 is shown cut away to reveal conductive material 700. A region of the conductive material is interfaced with the thermoelectric module 103. Conductive material 700 is formed by a plurality of fingers 713 extending from a central portion 713. Fingers 713 are configured to wrap around a portion of arm 101 in the region of the elbow joint. A cutout section is provided 701 to allow the elbow joint to move freely whilst allowing contact to be maintained between the conductive material and the skin of the arm.

Control means 703 is positioned remote from the thermoelectric module 103 and/or strap 102. According to further specific implementations of the present invention, control means 703 may be mounted at the thermoelectric, module 103 and/or strap 102.

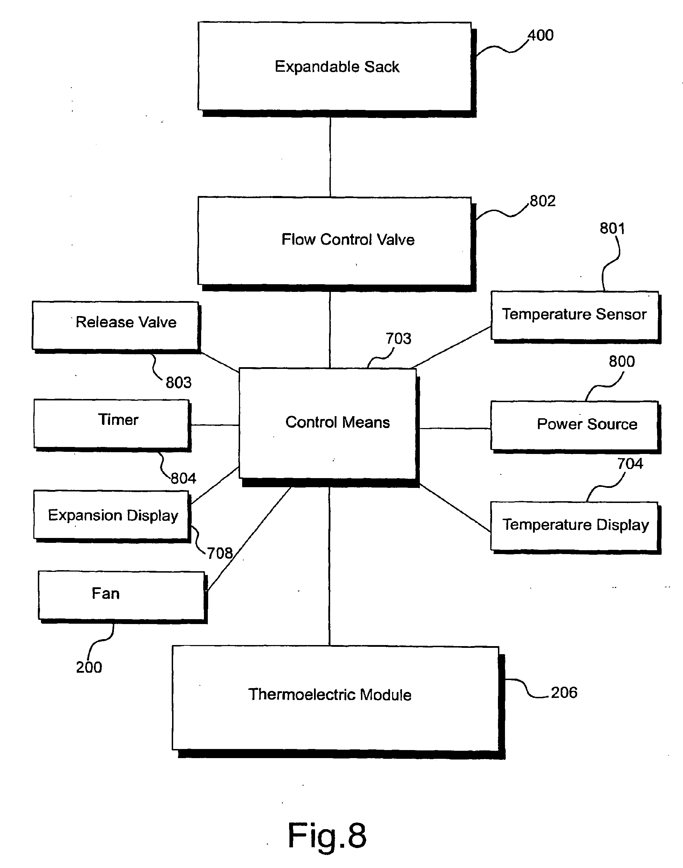

Figure 8 herein illustrates schematically selected components of the present invention. Means 702 allow communication between the control means 703 and the various components of the present invention including thermoelectric module 206, power source 800, temperature sensor 801, flow control valve 802, release valve 803, timer 804 and fan 200. Means 702 may comprise conventional electric wiring and/or fluid supply tubing connected to expandable sack 400.

According to further specific implementations of the present invention a fluid reservoir (not shown) may be positioned at strap 102 and/or layer 500, the reservoir providing a source of fluid for introduction into the expandable sack 400. Optionally, the fluid reservoir may be located remote from expandable sack 400 at the control means 703.

According to the specific implementation of the present invention fluid control valve 802 comprises a suitable solenoid valve configured to control the passage of air from an air intake (not shown) to the expandable sack 400. A release valve 803 is also provided to allow air to be released from sack 400.

One or a plurality of temperature sensors 801 are positioned at Peltier cell 206 and/or selected regions of the conductive material 209, 305. A temperature display 704 is configured to display a pre-set temperature of the Peltier cell and/or conductive material in addition to displaying the operative temperature of the Peltier cell and/or conductive material. Interface means are provided 705, 706 to allow a user to decrease or increase, respectively, a pre-set temperature of the Peltier cell.

Expansion display 708 is configured to display a relative magnitude of expansion of expandable sack 400. Additionally, means are provided 709, 710 to enable a user to set the desired amount of expansion of sack 400 and the corresponding degree of compressive force.

Additional means 707 is provided at control means 703 enabling the device to be turned on and off. Further means may also be provided 711, 712 allowing a user to activate the massaging effect provided by sack 400 via regulation of the fluid flow into and out of sack 400 using flow control valve 802. Means 711, 712 may be used to control timer 804 whereby the device may be pre-set for a desired operative time interval providing a cooling, heating effect and/or compressive force. Timer 804, in conjunction with release valve 803 provides a safety mechanism for regulating a predetermined volume of fluid introduced into sack 400 so as to prevent sack 400 over expanding and being damaged.

Control means 703 and the selected operative components of the present invention are powered by power source 800. Power source 800 may comprise at least one battery and/or means for interfacing with the mains electricity supply.

## Claims

1. A device configured to provide a heating and a cooling effect to a region of a human or animal, said device comprising:
at least one thermoelectric module capable of generating heat;
and said thermoelectric module also capable of providing a cooling effect;
at least one solid conductive material in contact with said thermoelectric module,
wherein said conductive material is capable of positioning in contact with said region of said human or animal and configured to transfer said heat and said cooling effect generated by said thermoelectric module to said human or animal;
an expandable sack, said sack comprising a plurality of compartments, said sack capable of generating a compressive force to said region of said human or animal; arid
means for securing said conductive material and said expandable sack in contact with said region of said human or animal;
wherein said plurality of compartments are each independently controllable such that the pressure in one compartment can be configured to be different to the pressure in another said compartment; and
the device further comprises means for regulating the amount of fluid introduced into said sack so as to provide a massaging action to said human or animal.

2. The device as claimed in claim 1 further comprising control means configured to control said heating and cooling effect provided by said thermoelectric module and an expansion of said expandable sack.

3. The device as claimed in claim 2 wherein said control means is positioned remote from said thermoelectric module.

4. The device as claimed in claims 2 or 3 further comprising a bladder positioned about said conductive material, said bladder capable of housing a conductive liquid.

5. The device as claimed in claim 4 wherein said bladder is an expandable bladder, said control means configured to control an amount of said liquid in said bladder wherein said bladder is capable of generating a compressive force to said region of said human or animal.

6. The device as claimed in claim 4 wherein said liquid is a gel.

7. The device as claimed in any one of claims 4 to 6 wherein said bladder is divided into a plurality of channels, said channels being in a fluid communication with one another.

8. The device as claimed in any one of claims 4 to 6 wherein said bladder is divided into a network of compartments, said compartments being in fluid communication with one another.

9. The device as claimed in any preceding claim wherein said expandable sack is capable of being expanded by a fluid introduced into said expandable sack.

10. The device as claimed in any preceding claim wherein said expandable sack is divided into a plurality of channels, said channels being in fluid communication with one another.

11. The device as claimed in any one of claims 1 to 9 wherein said expandable sack is divided into a network of compartments, said compartments being in fluid communication with one another.

12. The device as claimed in any preceding claim wherein said expandable sack is an inflatable sack configured to be inflated by introduction of air into said inflatable sack.

13. The device as claimed in any preceding claim wherein said conductive material is a metal.

14. The device as claimed in any preceding claim wherein said conductive material comprise at least two separate pieces of conductive material joined together and movable relative to one another.

15. The device as claimed in claim 14 wherein said pieces of conductive material are pivotally mounted to one another.

16. The device as claimed in claim 14 wherein said pieces of conductive material are slidably mounted to one another.

17. The device as claimed in any one of claims 14 to 16 wherein said pieces of conductive material are detachably joined together.

18. The device as claimed in any one of claims 14 to 17 wherein said conductive material is curved.

19. The device as claimed in any one of claims 14 to 18 wherein said conductive material comprises at least one bent or curved region.

20. The device as claimed in any one of claims 14 to 18 wherein said conductive material comprises a plurality of bent or curved regions.

21. The device as claimed in any preceding claim wherein a portion of said conductive material is enclosed by a fabric material.

22. The device as claimed in any preceding claim wherein a portion of said conductive material is covered by a foam material.

23. The device as claimed in any preceding claim wherein a portion of said conductive material is covered by a polymer material.

24. The device as claimed in any preceding claim wherein said conductive material is a metallic mesh.

25. The device as claimed in any preceding claim wherein said conductive material comprises a non-uniform thickness.

26. The device as claimed in any preceding claim wherein said conductive material is copper.

27. The device as claimed in any preceding claim wherein said means for securing said conductive material is a wrap configured for positioning around a body part or limb of said human or animal.

28. The device as claimed in claim 27 wherein said wrap is elasticated.

29. The device as claimed in any preceding claim further comprising heat dissipation means positioned at said thermoelectric module, said heat dissipation means configured to dissipate heat from said thermoelectric module.

30. The device as claimed in any preceding claim further comprising an insulating material positioned at said conductive material.

31. The device as claimed in any preceding claim further comprising a temperature sensor positioned at said thermoelectric module or said conductive material.

32. The device as claimed in claim 31 further comprising temperature display means being connected to said temperature sensor.

33. The device as claimed in any preceding claim wherein said control means is configured for the adjustable regulation of an amount of fluid introduced into said expandable sack so as to provide a massaging action to said human or animal when said device is secured to said region of said human or animal.

34. The device as claimed in claim 33 wherein said regulation of said amount of fluid introduced into said expandable sack is provided by a solenoid valve.

35. The device as claimed in any preceding claim wherein said thermoelectric module is a Peltier module.

## Patentansprüche

1. Vorrichtung, die so konfiguriert ist, dass sie eine Wärm- und eine Kühlwirkung in einer Region eines Menschen oder eines Tieres erzeugt, wobei die genannte Vorrichtung Folgendes umfasst:
wenigstens ein thermoelektrisches Modul, das Wärme erzeugen kann,
wobei das genannte thermoelektrische Modul auch eine Kühlwirkung erzeugen kann,
wenigstens ein festes leitendes Material in Kontakt mit dem genannten thermoelektrischen Modul, wobei das genannte leitende Material mit der genannten Region des genannten Menschen oder Tieres in Kontakt gebracht werden kann und so konfiguriert ist, dass die durch das genannte thermoelektrische Modul erzeugte genannte Wärm- und Kühlwirkung zu dem genannten Menschen oder Tier übertragen wird,
einen ausdehnbaren Beutel, wobei der genannte Beutel mehrere Fächer umfasst, wobei der genannte Beutel eine Druckkraft auf die genannte Region des genannten Menschen oder Tieres erzeugen kann,
ein Mittel zum Befestigen des genannten leitenden Materials und des genannten ausdehnbaren Beutels in Kontakt mit der genannten Region des genannten Menschen oder Tieres,
wobei die genannten mehreren Fächer jeweils unabhängig regulierbar sind, so dass der Druck in einem Fach so konfiguriert sein kann, dass er sich von dem Druck in einem anderen der genannten Fächer unterscheidet, und
ein Mittel zum Regulieren der in den genannten Beutel eingeleiteten Fluidmenge, um eine Massagewirkung beim genannten Menschen oder Tier zu erzeugen.

2. Vorrichtung nach Anspruch 1, die ferner ein Reguliermittel umfasst, das so konfiguriert ist, dass es die von dem genannten thermoelektrischen Modul erzeugte genannte Wärm- und Kühlwirkung und eine Ausdehnung des genannten ausdehnbaren Beutels reguliert.

3. Vorrichtung nach Anspruch 2, wobei das genannte Reguliermittel fern von dem genannten thermoelektrischen Modul positioniert ist.

4. Vorrichtung nach Anspruch 2 oder 3, die ferner eine um das genannte leitende Material positionierte Blase umfasst, wobei die genannte Blase eine leitende Flüssigkeit aufnehmen kann.

5. Vorrichtung nach Anspruch 4, wobei die genannte Blase eine ausdehnbare Blase ist, wobei das genannte Reguliermittel so konfiguriert ist, dass es eine Menge der genannten Flüssigkeit in der genannten Blase reguliert, wobei die genannte Blase eine Druckkraft auf die genannte Region des genannten Menschen oder Tieres erzeugen kann.

6. Vorrichtung nach Anspruch 4, wobei die genannte Flüssigkeit ein Gel ist.

7. Vorrichtung nach einem der Ansprüche 4 bis 6, wobei die genannte Blase in mehrere Kanäle unterteilt ist, wobei die genannten Kanäle in Fluidverbindung miteinander sind.

8. Vorrichtung nach einem der Ansprüche 4 bis 6, wobei die genannte Blase in ein Netz von Fächern unterteilt ist, wobei die genannten Fächer in Fluidverbindung miteinander sind.

9. Vorrichtung nach einem der vorherigen Ansprüche, wobei der genannte ausdehnbare Beutel durch ein in den genannten ausdehnbaren Beutel eingeführtes Fluid ausgedehnt werden kann.

10. Vorrichtung nach einem der vorherigen Ansprüche, wobei der genannte ausdehnbare Beutel in mehrere Kanäle unterteilt ist, wobei die genannten Kanäle in Fluidverbindung miteinander sind.

11. Vorrichtung nach einem der Ansprüche 1 bis 9, wobei der genannte ausdehnbare Beutel in ein Netz von Fächern unterteilt ist, wobei die genannten Fächer in Fluidverbindung miteinander sind.

12. Vorrichtung nach einem der vorherigen Ansprüche, wobei der genannte ausdehnbare Beutel ein aufblasbarer Beutel ist, der so konfiguriert ist, dass er durch Einleiten von Luft in den genannten aufblasbaren Beutel aufgeblasen werden kann.

13. Vorrichtung nach einem der vorherigen Ansprüche, wobei das genannte leitende Material ein Metall ist.

14. Vorrichtung nach einem der vorherigen Ansprüche, wobei das genannte leitende Material wenigstens zwei getrennte Stücke leitendes Material umfasst, die miteinander verbunden und relativ zueinander beweglich sind.

15. Vorrichtung nach Anspruch 14, wobei die genannten leitenden Materialstücke drehbar aneinander befestigt sind.

16. Vorrichtung nach Anspruch 14, wobei die genannten leitenden Materialstücke verschiebbar aneinander befestigt sind.

17. Vorrichtung nach einem der Ansprüche 14 bis 16, wobei die genannten leitenden Materialstücke lösbar miteinander verbunden sind.

18. Vorrichtung nach einem der Ansprüche 14 bis 17, wobei das genannte leitende Material gekrümmt ist.

19. Vorrichtung nach einem der Ansprüche 14 bis 18, wobei das genannte leitende Material wenigstens eine gebogene oder gekrümmte Region hat.

20. Vorrichtung nach einem der Ansprüche 14 bis 18, wobei das genannte leitende Material mehrere gebogene oder gekrümmte Regionen hat.

21. Vorrichtung nach einem der vorherigen Ansprüche, wobei ein Abschnitt des genannten leitenden Materials durch ein Gewebematerial umschlossen ist.

22. Vorrichtung nach einem der vorherigen Ansprüche, wobei ein Abschnitt des genannten leitenden Materials durch ein Schaumstoffmaterial bedeckt ist.

23. Vorrichtung nach einem der vorherigen Ansprüche, wobei ein Abschnitt des genannten leitenden Materials durch ein Polymermaterial bedeckt ist.

24. Vorrichtung nach einem der vorherigen Ansprüche, wobei das genannte leitende Material ein Metallgeflecht ist.

25. Vorrichtung nach einem der vorherigen Ansprüche, wobei das genannte leitende Material eine nicht einheitliche Dicke hat.

26. Vorrichtung nach einem der vorherigen Ansprüche, wobei das genannte leitende Material Kupfer ist.

27. Vorrichtung nach einem der vorherigen Ansprüche, wobei das genannte Mittel zum Befestigen des genannten leitenden Materials ein Wickel ist, der so konfiguriert ist, dass er um einen Körperteil oder eine Gliedmaße des genannten Menschen oder Tieres gelegt werden kann.

28. Vorrichtung nach Anspruch 27, wobei der genannte Wickel elastisch ist.

29. Vorrichtung nach einem der vorherigen Ansprüche, die ferner ein Wärmeabfuhrmittel umfasst, das an dem genannten thermoelektrischen Modul positioniert ist, wobei das genannte Wärmeabfuhrmittel so konfiguriert ist, dass es Wärme von dem genannten thermoelektrischen Modul abführt.

30. Vorrichtung nach einem der vorherigen Ansprüche, die ferner ein Isoliermaterial umfasst, das an dem genannten leitenden Material positioniert ist.

31. Vorrichtung nach einem der vorherigen Ansprüche, die ferner einen Temperatursensor umfasst, der an dem genannten thermoelektrischen Modul oder dem genannten leitenden Material positioniert ist.

32. Vorrichtung nach Anspruch 31, die ferner ein Temperaturanzeigemittel umfasst, das mit dem genannten Temperatursensor verbunden ist.

33. Vorrichtung nach einem der vorherigen Ansprüche, wobei das genannte Reguliermittel so konfiguriert ist, dass eine in den genannten ausdehnbaren Beutel eingeleitete Fluidmenge justierbar geregelt wird, um eine Massagewirkung bei dem genannten Menschen oder Tier zu erzeugen, wenn die genannte Vorrichtung an der genannten Region des genannten Menschen oder Tieres befestigt ist.

34. Vorrichtung nach Anspruch 33, wobei die genannte Regelung der in den genannten ausdehnbaren Beutel eingeleiteten genannten Fluidmenge durch ein Magnetventil erbracht wird.

35. Vorrichtung nach einem der vorherigen Ansprüche, wobei das genannte thermoelektrische Modul ein Peltier-Modul ist.

## Revendications

1. Appareil apte à fournir un effet chauffant et un effet refroidissant à une région d'un humain ou d'un animal, ledit appareil comprenant :
au moins un module thermoélectrique apte à produire de la chaleur :
et ledit module thermoélectrique étant également apte à produire un effet refroidissant ;
au moins un matériau conducteur solide au contact dudit module thermoélectrique, dans lequel ledit matériau conducteur est apte à être disposé au contact de ladite région dudit humain ou animal et apte à transférer ladite chaleur et ledit effet refroidissant produit par ledit module thermoélectrique audit humain ou animal ;
un sac expansible, ledit sac comprenant une pluralité de compartiments, ledit sac étant apte à exercer une force compressive sur ladite région dudit humain ou animal ; et
des moyens pour attacher ledit matériau conducteur et ledit sac expansible au contact de ladite région dudit humain ou animal ; et
des moyens pour réguler le montant de fluide introduit dans ledit sac, de sorte à fournir une action de massage audit humain ou animal ;
dans lequel
ladite pluralité de compartiments sont chacun indépendamment contrôlable, de sorte que la pression dans un compartiment puisse être configurée pour être différente par rapport à la pression dans un autre dit compartiment,

2. Appareil selon la revendication 1, comprenant en outre des moyens de contrôle aptes à contrôler ledit effet chauffant et refroidissant fourni par ledit module thermoélectrique et une expansion dudit sac expansible.

3. Appareil selon la revendication 2, dans lequel lesdits moyens de contrôle sont disposés à distance dudit module thermoélectrique.

4. Appareil selon la revendication 2 ou 3, comprenant en outre une vessie disposée autour dudit matériau conducteur, ladite vessie étant apte à loger un liquide conducteur.

5. Appareil selon la revendication 4, dans lequel ladite vessie est une vessie expansible, lesdits moyens de contrôle étant aptes à contrôler un montant dudit liquide dans ladite vessie, ladite vessie étant apte à exercer une force compressible sur ladite dudit région humain ou animal.

6. Appareil selon la revendication 4, dans lequel ledit liquide est un gel.

7. Appareil selon l'une des revendications 4 à 6, dans lequel ladite vessie est divisée en une pluralité des canaux, lesdits canaux étant en communication fluidique les uns avec les autres.

8. Appareil selon l'une des revendications 4 à 6, dans lequel ladite vessie est divisée en un réseau de compartiments, lesdits compartiments étant en communication fluidique les uns avec les autres.

9. Appareil selon l'une des revendications précédentes, dans lequel ledit sac expansible est apte à expansion au moyen d'un fluide introduit dans ledit sac expansible.

10. Appareil selon l'une des revendications précédentes, dans lequel ladite vessie est divisée en une pluralité des canaux, lesdits canaux étant en communication fluidique les uns avec les autres.

11. Appareil selon l'une des revendications précédentes, dans lequel ladite vessie est divisée en un réseau de compartiments, lesdits compartiments étant en communication fluidique les uns avec les autres.

12. Appareil selon l'une des revendications précédentes, dans lequel ledit sac expansible est un sac gonflable apte à être gonflé par l'introduction d'air dans ledit sac gonflable.

13. Appareil selon l'une des revendications précédentes, dans lequel ledit matériau conducteur est un métal.

14. Appareil selon l'une des revendications précédentes, dans lequel ledit matériau conducteur comprend au moins deux pièces distinctes de matériau conducteur, jointes ensemble et déplaçables l'une par rapport à l'autre.

15. Appareil selon la revendication 14, dans lequel lesdites pièces de matériau conducteur sont montées l'une à l'autre de façon pivotante.

16. Appareil selon la revendication 14, dans lequel lesdites pièces de matériau conducteur sont montées l'une à l'autre de façon coulissante.

17. Appareil selon l'une des revendications 14 à 16, dans lequel lesdites pièces de matériau conducteur sont jointes ensemble de façon détachable.

18. Appareil selon l'une des revendications 14 à 17, dans lequel ledit matériau conducteur est courbe.

19. Appareil selon l'une des revendications 14 à 18, dans lequel ledit matériau conducteur comprend au moins une région cintrée ou courbe.

20. Appareil selon l'une des revendications 14 à 18, dans lequel ledit matériau conducteur comprend une pluralité de régions cintrées ou courbes.

21. Appareil selon l'une des revendications précédentes, dans lequel une portion dudit matériau conducteur est enveloppée par un matériau textile.

22. Appareil selon l'une des revendications précédentes, dans lequel une portion dudit matériau conducteur est enveloppée par un matériau mousseux.

23. Appareil selon l'une des revendications précédentes, dans lequel une portion dudit matériau conducteur est enveloppée par un matériau polymère.

24. Appareil selon l'une des revendications précédentes, dans lequel ledit matériau conducteur est une maille métallique.

25. Appareil selon l'une des revendications précédentes, dans lequel ledit matériau conducteur possède une épaisseur non - uniforme.

26. Appareil selon l'une des revendications précédentes, dans lequel ledit matériau conducteur est du cuivre.

27. Appareil selon l'une des revendications précédentes, dans lequel lesdits moyens pour attacher ledit matériau conducteur sont une enveloppe apte à disposition autour d'une partie du corps ou d'un membre dudit humain ou animal.

28. Appareil selon la revendication 27, dans lequel ladite enveloppe est élastique.

29. Appareil selon l'une des revendications précédentes, comprenant en outre des moyens de dissipation de chaleur disposés au niveau dudit module thermoélectrique, lesdits moyens de dissipation de chaleur étant aptes à dissiper de la chaleur provenant dudit module thermoélectrique.

30. Appareil selon l'une des revendications précédentes, comprenant en outre un matériau isolant disposé au niveau dudit matériau conducteur.

31. Appareil selon l'une des revendications précédentes, comprenant en outre un détecteur de température disposé au niveau dudit module thermoélectrique ou dudit matériau conducteur.

32. Appareil selon la revendication 31, comprenant en outre des moyens d'affichage de température, qui sont connectés audit détecteur de température.

33. Appareil selon l'une des revendications précédentes, dans lequel lesdits moyens de contrôle sont aptes à réguler de façon ajustable le montant d'un fluide introduit dans ledit expansible, de sorte à fournir une action de massage audit humain ou animal lorsque ledit appareil est attaché à ladite région dudit humain ou animal.

34. Appareil selon la revendication 33, dans lequel ladite régulation dudit montant de fluide introduit dans ledit sac expansible est fournie par une soupape électromagnétique.

35. Appareil selon l'une des revendications précédentes, dans lequel ledit module thermoélectrique est un module Peltier.
